# EUROPÄISCHE PATENTSCHRIFT

(11) **EP 3 127 645 B1**
(45) Veröffentlichungstag und Bekanntmachung des Hinweises auf die Patenterteilung: **06.11.2019**
(21) Anmeldenummer: 16001654.9
(22) Anmeldetag: 27.07.2016
(51) Int. Cl.: B23K 1/00, B23K 1/19, A61B 1/267, B23K 35/30, B23K 103/04

(54) **VERFAHREN ZUM FÜGEN ZWEIER BAUTEILE EINES INVASIVEN MEDIZINISCHEN INSTRUMENTS UND INVASIVES MEDIZINISCHES INSTRUMENT**
METHOD FOR JOINING TWO COMPONENTS OF AN INVASIVE MEDICAL INSTRUMENT AND INVASIVE MEDICAL INSTRUMENT
PROCÉDÉ D'ASSEMBLAGE DE DEUX COMPOSANTS D'UN INSTRUMENT MÉDICAL INVASIF ET INSTRUMENT MÉDICAL INVASIF

(30) Priorität: 04.08.2015 DE 102015009858
(43) Veröffentlichungstag der Anmeldung: 08.02.2017
(73) Patentinhaber: Karl Storz SE & Co. KG, 78532 Tuttlingen (DE)
(72) Erfinder: Krause, Bernd, D-78532 Tuttlingen (DE)
(74) Vertreter: Fugmann, Winfried

(56) Entgegenhaltungen:
- DE-C2- 3 119 725
- Oerlikon: "An Introduction to Brazing - Fundamentals, Materials, Processing An Introduction to Brazing - Issue 4", , 30. September 2014 (2014-09-30), Seiten 1-24, XP055291435, Gefunden im Internet: URL:https://www.oerlikon.com/ecomaXL/files /oerlikon_Introduction_to_Brazing_EN4.pdf& download=1 [gefunden am 2016-07-26]
- Ina M. Hoyer: "Beitrag zur Entwicklung von Hochtemperaturloten auf Eisenbasis", , 23. August 2005 (2005-08-23), Seiten 1-117, XP055316593, Gefunden im Internet: URL:http://www.qucosa.de/fileadmin/data/qu cosa/documents/5069/data/Dissertation_Hoye r.pdf [gefunden am 2016-11-04]
- Katja Ulrike Heintzenberg: "Vergleich der mechanischen Festigkeiten gelöteter und lasergeschweißter Prüfkörper aus einer Palladiumbasis-Legierung nach chemischer Belastung", , 13. September 2002 (2002-09-13), XP055316640, Gefunden im Internet: URL:http://webdoc.sub.gwdg.de/ebook/diss/2 003/fu-berlin/2002/148/03_schrifttum_02080 8.pdf [gefunden am 2016-11-04]

## Beschreibung

Die vorliegende Erfindung betrifft ein Verfahren zum Fügen zweier Bauteile eines invasiven medizinischen Instruments, sowie ein invasives medizinisches Instrument (siehe, z.B., DE 31 19725 C2).

Medizinische Instrumente, insbesondere invasive medizinische Instrumente, die zum Eindringen in einen menschlichen oder tierischen Körper bestimmt sind, unterliegen besonderen Anforderungen. Häufig werden derartige Instrumente aus Gründen der mechanischen und thermischen Belastbarkeit unter Verwendung metallischer Werkstoffe hergestellt. Insbesondere die Materialien, die die äußeren Oberflächen des Instruments bilden, müssen biokompatibel sein. Sofern das medizinische Instrument aus konstruktiven Gründen aus einer Mehrzahl einzelner Bauteile zusammengesetzt ist, ist eine dauerhafte Verbindung der Bauteile miteinander erforderlich. Die Verbindung muss dabei sowohl die notwendige Festigkeit als auch eine ausreichende Beständigkeit gegen chemische und thermische Einflüsse aufweisen und die Anforderungen der Biokompatibilität erfüllen. Sofern durch die miteinander verbundenen Bauteile ein Hohlraum gebildet wird, in dem empfindliche oder nicht-biokompatible Komponenten aufgenommen sind, muss die Verbindung der Bauteile außerdem fluiddicht sein, um beispielsweise ein Eindringen von Körper- oder Reinigungsflüssigkeit in den Hohlraum zu vermeiden. Diese Anforderungen gelten in erhöhtem Maße für wiederverwendbare medizinische Instrumente, die zur Reinigung und Sterilisation geeignet sein müssen, wobei sie chemisch aggressiven Substanzen sowie beim Autoklavieren einer erhöhten Temperatur und einem erhöhten Druck ausgesetzt sind.

Aus DE 31 19 725 C2 ist ein Laryngoskopspatel bekannt, der durch zwei einander übergreifend aneinander angesetzte L-förmige Längsprofile gebildet wird, die miteinander verlötet sind. Dabei sind an den Anlagekanten der L-förmigen Längsprofile vorspringende Warzen ausgebildet, die in im anderen Längsprofil ausgebildete Wellen eingreifen, wodurch sich nach dem Anlegen der Längsprofile ein gegenseitiger Halt ergibt. Die Profile werden dann durch Widerstandsschweißung aneinander befestigt und die verbleibenden Spalte durch Hartlöten verschlossen.

Gemäß DE 33 17 831 C2 weist ein Spatel eines Laryngoskops ein Z-Profilstück und ein L-Profilstück auf, wobei die Verbindung zwischen einem ersten Schenkel des Z-Profilstücks und einer Mittelstegwand mit einem kürzeren Schenkel des L-Profilstücks als Schweißnaht ausgeführt ist, die anschließend feingeschliffen und feinpoliert wird. Die Verbindungsstelle eines zweiten Schenkels des Z-Profilstücks mit dem längeren Schenkel des L-Profilstücks ist gemäß einem Lötverfahren mit so viel Lötgut hergestellt, dass ein deutlicher Abrundungsradius entsteht und eine weitgehend lückenfreie Oberfläche gewährleistet ist.

In EP 2 151 185 B1 ist ein Laryngoskopspatel mit einem Spatelblatt offenbart, wobei zum Herstellen des Spatelblatts eine Ober- und eine Unterschale bereitgestellt werden, die jeweils im Querschnitt einen ersten Abschnitt und zwei zueinander entgegengesetzt verlaufende zweite und dritte Abschnitte aufweisen, die zweiten und die dritten Abschnitte der Ober- und der Unterschale flächig aufeinandergelegt werden und die Ober- und die Unterschale miteinander durch Fügen der zweiten und dritten Abschnitte fest verbunden werden. Dabei können die Ober- und die Unterschale miteinander an einer Kontaktkante, die jeweils aneinandergrenzende Kanten der zweiten und dritten Abschnitte miteinander bilden, umlaufend verlötet werden.

Zum Verbinden der Einzelteile eines Laryngoskopspatels mittels Löten wird häufig ein Kupferbasislot verwendet. Da Kupfer nicht biokompatibel ist, werden derartig hergestellte Spatel üblicherweise zusätzlich verschweißt und verchromt, was einen zusätzlichen Fertigungsaufwand verursacht. Weiterhin ist die Verwendung eines Nickelbasislots bekannt. Aufgrund der Fließeigenschaften des Nickelbasislots und um eine gleichmäßige Oberfläche zu erhalten, werden beim Löten Antiflussmittel eingesetzt, die jedoch einen Ofen, in dem das Löten stattfindet und in dem das Antiflussmittel verdampft, beschädigen können. Darüber hinaus kann durch Nickelbasislot nur ein geringes Spaltmaß überbrückt werden, es entsteht oft eine raue oder porige Oberfläche und zudem ist das Überschweißen einer mit Nickelbasislot erzeugten Lötnaht nicht ohne weiteres möglich. Andere stoffschlüssige Fügeverfahren, wie etwa das Laserschweißen, haben andererseits den Nachteil, dass derart erzeugte Schweißnähte, insbesondere längere Schweißnähte, häufig nicht dampfdicht sind; eine dampfdichte Ausführung von Schweißnähten ist jedoch für ein autoklavierbares medizinisches Instrument, das einen Hohlraum mit empfindlichen elektronischen und/oder optischen Komponenten aufweist, erforderlich.

Aus DE 31 45 944 C2 ist eine Lotlegierung bekannt, die aus 25 bis 35% Eisen, 15 bis 25% Chrom, 3 bis 6% Silizium, 1 bis 4% Molybdän, Rest Kobalt besteht, die zum Löten von Teilen für Zahnprothesen verwendet wurde, die aus einer Kobalt-Chrom-Legierung bestanden.

Es ist Aufgabe der vorliegenden Erfindung, ein Verfahren zum Fügen mindestens zweier Bauteile eines invasiven medizinischen Instruments, insbesondere eines Laryngoskopspatels, sowie ein mit einem entsprechenden Verfahren hergestelltes invasives medizinisches Instrument anzugeben, wobei die oben genannten Nachteile möglichst vermieden werden. Insbesondere ist es Aufgabe der Erfindung, ein Verfahren zum Fügen mindestens zweier Bauteile eines invasiven medizinischen Instruments mittels Hartlöten anzugeben, wobei die mindestens zwei Bauteile auf einfache und kostengünstige Weise fest und dauerhaft miteinander verbunden werden können und wobei die erzeugte Lötnaht eine korrosionsbeständige Oberfläche aufweist und biokompatibel ist.

Diese Aufgabe wird durch ein Verfahren gemäß Anspruch 1, durch Verwendung eines Eisenbasislots gemäß Anspruch 8 sowie durch ein invasives medizinisches Instrument gemäß Anspruch 9 gelöst.

Vorteilhafte Weiterbildungen der Erfindung ergeben sich aus den Unteransprüchen.

Ein erfindungsgemäßes Verfahren dient zum Fügen, d.h. zum dauerhaften Verbinden mindestens zweier Bauteile eines invasiven medizinischen Instruments. Die Bauteile sind vorzugsweise metallische Bauteile, insbesondere Bauteile aus Edelstahl, die Bauteile können aber auch zumindest teilweise aus einem anderen für die Verwendung in einem medizinischen Instrument und für das Lötverfahren geeigneten, vorzugsweise metallischen Werkstoff bestehen.

Zunächst werden die mindestens zwei miteinander zu verbindenden Bauteile bereitgestellt. Diese sind derart ausgebildet, dass sie jeweils mindestens einen Fügebereich aufweisen, wobei die Fügebereiche der mindestens zwei miteinander zu verbindenden Bauteile einander derart zugeordnet sind, dass sie zum Ausbilden eines Lötspalts zusammenwirken können. Die Fügebereiche weisen somit eine solche Form auf, dass, wenn die Bauteile entsprechend zueinander gehalten werden, zwischen den Fügebereichen ein Spalt mit einer zum Löten geeigneten Spaltweite und Spaltfläche gebildet wird. Die mindestens zwei miteinander zu verbindenden Bauteile werden sodann derart zueinander angeordnet und gehalten, dass zwischen den einander zugeordneten Fügebereichen der Bauteile mindestens ein Lötspalt gebildet wird. Diejenigen Bereiche der Oberflächen der Bauteile, die die Fügebereiche bilden, stehen somit einander in einem geringen Abstand gegenüber und bilden dadurch den mindestens einen Lötspalt. Hierfür können die Bauteile beispielsweise formschlüssig, reibschlüssig oder stoffschlüssig aneinander gehalten sein oder auch in einer entsprechenden Vorrichtung gehalten werden.

Weiter wird erfindungsgemäß das Lotmaterial in einer zum Löten geeigneten Weise angeordnet, d.h. das Lotmaterial wird in Bezug auf die mindestens zwei miteinander zu verbindenden Bauteile derart angeordnet, dass es nach dem Aufschmelzen beim Löten den mindestens einen Lötspalt zumindest teilweise ausfüllt. Hierfür kann das Lotmaterial an dem mindestens einen Lötspalt oder auch beispielsweise in Kapillarverbindung mit dem mindestens einen Lötspalt angeordnet werden, insbesondere an jeweils einem der den mindestens einen Lötspalt bildenden, miteinander zu verbindenden Bauteile oder auch an mehreren Bauteilen, zwischen denen der mindestens eine Lötspalt gebildet wird. Das Lotmaterial kann auch bereits zu einem Zeitpunkt, bevor die mindestens zwei miteinander zu verbindenden Bauteile zum Ausbilden des Lötspalts gehalten werden, an mindestens einem der Bauteile angeordnet werden. Das Lotmaterial kann insbesondere in einer derart vorbestimmten Menge bereitgestellt und angeordnet werden, dass der mindestens eine Lötspalt ganz oder zumindest weitgehend ausgefüllt wird.

Die so erhaltene Anordnung aus den mindestens zwei Bauteilen des invasiven medizinischen Instruments und dem Lotmaterial, die im Folgenden auch als "Lotverbund" bezeichnet wird, wird sodann auf eine Löttemperatur erwärmt, d.h. insbesondere auf eine Temperatur, die mindestens der Schmelztemperatur des Lotmaterials entspricht, jedoch insbesondere unter einer Schmelztemperatur des Materials der mindestens zwei Bauteile liegt. Hierfür wird der Lotverbund insbesondere in einen entsprechenden Ofen eingebracht, wo der Lotverbund beispielsweise unter Vakuum oder Schutzgas auf die Löttemperatur erwärmt wird. Hierbei verflüssigt sich das Lotmaterial, wird durch Kapillarkräfte in den mindestens einen Lötspalt gezogen und füllt diesen ganz oder teilweise aus. Nachdem das Lot den mindestens einen Lötspalt ausgefüllt hat, wird die Anordnung abkühlen gelassen, bis das Lotmaterial erstarrt ist und die mindestens zwei Bauteile durch die Lötverbindung fest miteinander verbunden sind. Die mindestens zwei Bauteile sind somit insbesondere über eine durch das Eisenbasislot gebildete Lötnaht stoffschlüssig miteinander verbunden.

Erfindungsgemäß ist das Lotmaterial ein Eisenbasislot, d.h. ein Lotmaterial auf Eisenbasis. Ein Eisenbasislot enthält beispielsweise etwa 28 bis 35 Gewichts-% Eisen sowie weitere Bestandteile, die in geringeren Gewichtsanteilen als Eisen enthalten sind. Ein solches Eisenbasislot, das gemäß EN ISO 3677 als B-FeCrNiSiP-1027/1097 bezeichnet wird, wird beispielsweise von der Fa. Innobraze (Esslingen, DE) unter der Bezeichnung ML 7813/S angeboten.

Erfindungsgemäß ist erkannt worden, dass durch Verwendung von Eisenbasislot auf einfache und sichere Weise eine dauerhafte Verbindung zwischen den mindestens zwei Bauteilen geschaffen werden kann, wobei die Anforderungen, die an Verbindungen von Bauteilen medizinischer Instrumente gestellt sind, erfüllt werden können. So ist Eisenbasislot biokompatibel, rostfrei und weist günstigere Fließeigenschaften als Nickelbasislot auf. Weitere Schritte wie etwa das Verchromen sind daher nicht notwendig, und ebenso kann auf die Verwendung von Antiflussmittel verzichtet werden. Daher sind bei der Fertigung des medizinischen Instruments weniger Arbeitsschritte erforderlich, so dass eine schnellere und kostengünstige Herstellung ermöglicht wird.

Das erfindungsgemäße Verfahren kann weitere Schritte umfassen, etwa eine Vorbehandlung der miteinander zu verbindenden Bauteile bzw. der Fügebereiche. Vor oder nach der Herstellung der erfindungsgemäßen Lötverbindung können weitere Montageschritte stattfinden, die insbesondere die Herstellung der miteinander zu verbindenden Bauteile und die weitere Montage des medizinischen Instruments nach dem Abkühlen des Lotverbunds betreffen.

Vorzugsweise werden mit dem erfindungsgemäßen Verfahren mehr als zwei Bauteile miteinander verbunden. Dabei können beispielsweise jeweils zwei Bauteile zum Ausbilden eines Lötspalts gehalten und das Lotmaterial an dem Lötspalt angeordnet werden, wobei diese Schritte für alle durch Löten mittels Eisenbasislot zu verbindenden Bauteile ausgeführt und der entstehende Lotverbund sodann zum Durchführen des Lötvorgangs erwärmt wird. Es können aber auch zunächst alle durch Löten mittels Eisenbasislot zu verbindenden Bauteile entsprechend angeordnet und danach das Lotmaterial zum Ausfüllen der Lötspalte angeordnet werden, bevor der Lotverbund auf Löttemperatur erwärmt wird. Hierbei kann auch ausgenutzt werden, dass mehrere, insbesondere alle Lötverbindungen des medizinischen Instruments gleichzeitig bzw. in einem einzigen Erwärmungsschritt erzeugt werden können. Hierdurch kann das invasive medizinische Instrument mit einem Minimum an Fertigungsschritten hergestellt werden.

Gemäß einer bevorzugten Ausführungsform der Erfindung werden die mindestens zwei miteinander zu verbindenden Bauteile vor dem Erwärmen, insbesondere vor dem Anordnen des Lotmaterials an mindestens einem der Bauteile, mittels Laserschweißen miteinander verbunden, um den Lötspalt zwischen den Fügebereichen auszubilden. Vorzugsweise werden die mindestens zwei Bauteile nur an wenigen Stellen mittels Laser-Punktschweißen miteinander verbunden. Die Erzeugung einiger weniger Schweißpunkte ist mit einem geringen Aufwand möglich und gestattet es, einen Lotverbund zu schaffen, der ausreichend fest ist, um eine einfache Handhabung zu ermöglichen, und der die Einhaltung einer vorgegebenen Spaltweite des mindestens einen Lötspalts sicherstellt. Ferner kann dadurch beispielsweise auf eine Hilfsvorrichtung zum Halten der mindestens zwei Bauteile verzichtet werden. Dadurch kann die Herstellung des medizinischen Instruments weiter vereinfacht werden.

In bevorzugter Weise sind die mindestens zwei miteinander zu verbindenden Bauteile des medizinischen Instruments derart ausgebildet, dass zwischen diesen beim Anordnen zum Ausbilden des mindestens einen Lötspalts ein Hohlraum gebildet wird. Vorzugsweise wird das Eisenbasislot zum Ausfüllen des Lötspalts innerhalb des Hohlraums angeordnet. Hierfür können zunächst die mindestens zwei Bauteile zum Schaffen des Hohlraums gehalten werden und danach das Eisenbasislot in den Hohlraum eingebracht und insbesondere auf mindestens eine an den Lötspalt angrenzende Innenwand des Hohlraums aufgebracht werden, vorzugsweise an zwei an den Lötspalt angrenzende Innenwände. Es kann aber auch das Eisenbasislot auf mindestens eines der zu verbindenden Teile aufgebracht werden, bevor diese den Hohlraum bilden, und danach die mindestens zwei Teile zum Ausbilden des Hohlraums angeordnet werden. Beim Erwärmen auf die Löttemperatur dringt das Lot von innen in den Lötspalt ein und füllt diesen aus. Dadurch, dass das Lotmaterial dem Lötspalt von der Innenseite her zugeführt wird, kann auf einfache Weise eine zumindest auf der Außenseite saubere und glatte Ausführung der Lötnaht ermöglicht werden, ohne dass beispielsweise aufgeklebte Begrenzungen notwendig wären, um ein Zerfließen des Lots zu verhindern.

Vorzugsweise wird das Lotmaterial, das ein Eisenbasislot ist, als Lotpaste bereitgestellt, etwa als Metallpulver, das mit einem Bindemittel versetzt ist und ein pastöses Lotmaterial bildet. Insbesondere wird dieses in den zwischen den mindestens zwei Bauteilen gebildeten Hohlraum eingebracht. Hierfür kann beispielsweise ein Applikator verwendet werden, der eine Dosiereinrichtung und eine Spritzennadel umfasst, wobei die Dosiereinrichtung das Ausbringen einer vorbestimmten Menge des Lotmaterials gestattet und die Spritzennadel durch ihre Länge und ggf. Krümmung der Form und Größe des Hohlraums angepasst ist. Durch eine Öffnung des Hohlraums kann somit das pastöse Lotmaterial durch Betätigung der Dosiereinrichtung mit der Spitze der Spritzennadel auf mindestens eine Innenwand des Hohlraums aufgetragen werden, insbesondere an dem mindestens einen Lötspalt. Hierdurch wird auf einfache Weise das Anordnen einer gewünschten Menge des Lotmaterials zum Ausfüllen des Lötspalts und zum Erzeugen einer glatten Lötnaht ermöglicht.

In vorteilhafter Weise ist der Hohlraum nach dem Erwärmen durch mindestens eine Lötnaht abgeschlossen, die fluiddicht ausgebildet ist. Der Hohlraum kann in weiteren Teilbereichen durch weitere Mittel abgeschlossen sein. So kann der Hohlraum beispielsweise in langerstreckter Form ausgebildet sein, wobei die Seitenwände des Hohlraums durch die mindestens zwei Bauteile des medizinischen Instruments gebildet werden, die miteinander durch die mindestens eine fluiddichte Lötnaht verbunden sind. An den Stirnseiten des langerstreckten Hohlraums kann dieser beispielsweise durch die Bauteile selbst oder durch ein eingeklebtes oder eingelötetes Abschlusselement abgeschlossen sein. Zum Herstellen einer fluiddichten, insbesondere dampfdichten, Verbindung kann das Lotmaterial in einer solchen Menge und einer solchen Verteilung entlang des Lötspalts eingebracht werden, dass der Lötspalt lückenlos ausgefüllt wird. Insbesondere kann eine aufgrund des auszufüllenden Volumens des Lötspalts und ggf. entstehender Hohlkehlen der Lötnaht bestimmte Menge des Lotmaterials mit dem Applikator gleichmäßig entlang des Lötspalts aufgetragen werden. Dadurch kann erreicht werden, dass beim Ausführen des Lötvorgangs durch Aufschmelzen des Lotmaterials der Lötspalt praktisch vollständig mit dem Eisenbasislot ausgefüllt ist. Nach dem Abkühlen und Erstarren des Lots entsteht daher eine fluiddichte Lötnaht, die einen dampfdichten Abschluss des Hohlraums ermöglicht. Hierdurch kann es auf einfache und sichere Weise ermöglicht werden, den Hohlraum auch bei autoklavierbaren medizinischen Instrumenten zur Aufnahme von elektronischen und/oder optischen Komponenten zu nutzen, die gegen Eindringen von Dampf empfindlich sind.

In bevorzugter Weise wird nach dem Ausführen des Lötvorgangs, d.h. nachdem das Lotmaterial in den mindestens einen Lötspalt eingedrungen ist und diesen mindestens teilweise ausfüllt, und nach Abkühlen und Erstarren des Eisenbasislots die mindestens eine durch das Eisenbasislot gebildete Lötnaht überschweißt. Hierdurch kann die Festigkeit der Verbindung der mindestens zwei Bauteile weiter erhöht werden. Gemäß diesem Aspekt der Erfindung ist erkannt worden, dass eine durch Eisenbasislot gebildete Lötnaht sich einfach überschweißen lässt und beispielsweise nicht zur Rissbildung neigt.

Vorzugsweise wird die überschweißte Lötnaht geglättet, beispielsweise durch Schleifen und/oder Polieren. Hierdurch kann die Oberflächengüte verbessert werden, so dass insbesondere die Reinigung des medizinischen Instruments erleichtert wird.

Die Erfindung betrifft ferner die Verwendung eines Eisenbasislots zum Fügen mindestens zweier Bauteile eines invasiven medizinischen Instruments. Erfindungsgemäß ist erkannt worden, dass bei einem stoffschlüssigen Fügen mittels Hartlöten durch Verwendung eines Eisenbasislots auf besonders einfache und sichere Weise eine dauerhafte Verbindung zwischen den mindestens zwei Bauteilen geschaffen werden kann, wobei die Anforderungen, denen medizinische Instrumente unterliegen, sicher erfüllt werden können. Hierdurch wird eine besonders kostengünstige Fertigung etwa eines Laryngoskopspatels ermöglicht. Insbesondere kann bei der Verwendung von Eisenbasislot zum Fügen mindestens zweier Bauteile des medizinischen Instruments das oben beschriebene Verfahren angewendet werden.

Ein erfindungsgemäßes medizinisches Instrument umfasst mindestens zwei Bauteile, die gemäß dem zuvor beschriebenen Verfahren bzw. durch Hartlöten unter Verwendung von Eisenbasislot miteinander verbunden worden sind. Die mindestens zwei miteinander gefügten Bauteile des invasiven medizinischen Instruments sind somit durch mindestens eine Lötnaht miteinander verbunden, die gemäß dem oben beschriebenen Verfahren erzeugt bzw. unter Verwendung von Eisenbasislot erstellt worden ist. Vorzugsweise sind die mindestens zwei Bauteile zumindest in ihrem jeweiligen Fügebereich bzw. in ihren jeweiligen Fügebereichen aus Edelstahl hergestellt. Derartige Bauteile können mit dem erfindungsgemäßen Verfahren besonders fest und sicher miteinander verbunden werden und sind zudem für medizinische Anwendungen geeignet. Ein derartiges medizinisches Instrument, insbesondere ein Laryngoskopspatel, kann dadurch in besonders einfacher Weise zur Erfüllung der an medizinische Instrumente, insbesondere an invasive medizinische Instrumente, gestellten Anforderungen ausgebildet werden.

Vorzugsweise ist das medizinische Instrument als Laryngoskopspatel ausgebildet, der ein Grundblatt und ein Deckblatt aufweist, die mit dem erfindungsgemäßen Verfahren und somit über mindestens eine durch ein Eisenbasislot gebildete Lötnaht miteinander verbunden sind. Das Grundblatt und das Deckblatt schließen einen Hohlraum ein, der sich in Längsrichtung des Spatels erstreckt und der durch die mindestens eine mit dem erfindungsgemäßen Verfahren geschaffene Lötnaht zumindest einseitig fluiddicht begrenzt ist. So kann beispielsweise der Hohlraum seitlich durch das Grundblatt und das Deckblatt sowie die zwischen diesen gebildeten fluiddichten Lötnähte abgeschlossen sein, während in Längsrichtung an den Enden des Hohlraums Öffnungen bestehen können, die durch Abschlusselemente abgeschlossen sein können. In dem auf diese Weise dampfdicht abgeschlossenen Hohlraum können beispielsweise elektronische und/oder optische Bauelemente aufgenommen sein. Durch den fluiddichten Abschluss des Hohlraums kann es ermöglicht werden, dass der Laryngoskopspatel autoklavierbar ist.

Vorzugsweise weist das invasive medizinische Instrument mindestens ein weiteres Bauteil auf, das über mindestens eine weitere, nach dem oben beschriebenen Verfahren erzeugte, Lötnaht mit einem der mindestens zwei miteinander verbundenen Bauteile verbunden ist. Bei einem erfindungsgemäßen Laryngoskopspatel ist das mindestens eine weitere Bauteil beispielsweise ein an einem distalen Ende des Laryngoskopspatels angeordneter atraumatischer Wulst und/oder ein an einem proximalen Ende des Laryngoskopspatels angeordneter Spatelkopf, der eine mechanische Kupplung sowie ggf. elektrische und/oder optische Kupplungen zum Anschluss an entsprechende Elemente eines Handgriffs des Laryngoskops aufweisen kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 einen Laryngoskopspatel gemäß einem Ausführungsbeispiel der Erfindung in einer Schrägansicht;
Fig. 2 den Laryngoskopspatel gemäß Fig. 1 in einer längsseitigen Ansicht;
Fig. 3a und 3b einen Querschnitt durch den Laryngoskopspatel gemäß Fig. 1 und 2 vor und nach dem Ausführen des Lötvorgangs.

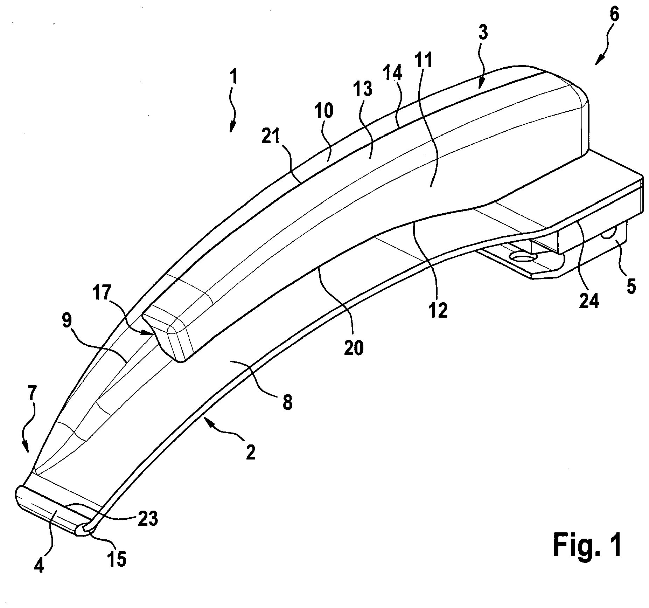

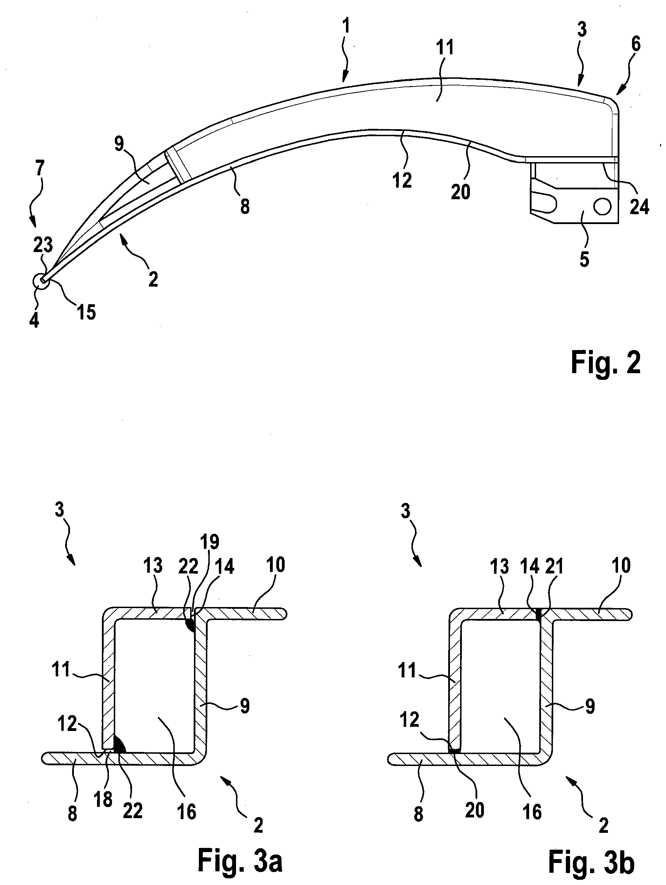

In den Figuren 1 und 2 ist ein Laryngoskopspatel 1 gemäß einem Ausführungsbeispiel der Erfindung in einer Schrägansicht sowie in einer Seitenansicht dargestellt. Der Laryngoskopspatel 1 umfasst vier Bauteile, die gemäß einem erfindungsgemäßen Verfahren miteinander verbunden sind, nämlich ein Grundblatt 2, ein Deckblatt 3, einen Wulst 4 und einen Spatelkopf 5. An den am benutzernahen (proximalen) Ende 6 des Laryngoskopspatels 1 angeordneten Spatelkopf 5 kann ein in den Figuren nicht dargestellter Handgriff angesetzt werden, mit dem der Laryngoskopspatel 1 bei einer Untersuchung gehalten werden kann. Der Laryngoskopspatel 1 kann in den Mund-Rachenraum eines Patienten eingeführt werden und weist hierfür eine an die anatomischen Gegebenheiten des Mund-Rachenraums angepasste abgeflachte, gebogene Form auf. Dabei ermöglicht der am benutzerfernen (distalen) Ende 7 des Laryngoskopspatels 1 angeordnete Wulst 4 ein leichtes und atraumatisches Einführen.

Das Grundblatt 2 weist einen unteren Schenkel 8 auf, der sich in Längsrichtung des Laryngoskopspatels 1 erstreckt und eine flächige, gekrümmte Unterseite und eine ebensolche Oberseite aufweist. In Querrichtung setzt an dem unteren Schenkel 8 ein in einem Winkel zu diesem stehender mittlerer Schenkel 9 an, an den wiederum ein oberer Schenkel 10 ansetzt, der sich in Querrichtung gesehen im Wesentlichen parallel zum unteren Schenkel 8 erstreckt. Der mittlere Schenkel 9 steht abschnittsweise näherungsweise senkrecht auf dem unteren Schenkel 8 und dem oberen Schenkel 10. Die Höhe des mittleren Schenkels 9 nimmt vom proximalen Ende 6 zum distalen Ende 7 hin insgesamt ab, wobei die Höhe des mittleren Schenkels 9 nahe dem proximalen Ende 6 ein Maximum erreicht und in der Nähe des distalen Endes 7 auf null zurückgeht. Im Querschnitt ist das Grundblatt somit im Bereich des proximalen Endes 6 des Laryngoskopspatels 1 sowie über den überwiegenden Teil seiner Längserstreckung näherungsweise Z-förmig ausgebildet und geht im Bereich des distalen Endes 7 des Laryngoskopspatels 1 in eine flache Querschnittsform über. Das Grundblatt 2 ist einstückig ausgebildet und besteht aus Edelstahl.

Das Deckblatt 3 des Laryngoskopspatels 1 umfasst einen ersten Schenkel 11, der sich in Längsrichtung des Laryngoskopspatels 1 erstreckt und näherungsweise senkrecht auf der Oberseite des unteren Schenkels 8 des Grundblatts 2 steht. Die untere Kante 12 des ersten Schenkels 11 ist an die Form der Oberseite des unteren Schenkels 8 des Grundblatts 2 angepasst. Im oberen Bereich des ersten Schenkels 11 schließt sich an diesen und näherungsweise senkrecht zu diesem stehend ein zweiter Schenkel 13 an, dessen Oberseite in die Oberseite des oberen Schenkels 10 des Grundblatts 2 übergeht und mit dieser eine im Wesentlichen durchgehende gekrümmte Fläche bildet. Die Seitenkante 14 des zweiten Schenkels 13 ist hierfür an den Verlauf des Übergangs zwischen dem mittleren Schenkel 9 und dem oberen Schenkel 10 des Grundblatts 2 angepasst. Das Deckblatt 3 weist eine näherungsweise L-förmige Querschnittsform auf, wobei die Höhe des ersten Schenkels 11 in der Nähe des distalen Endes 6 des Laryngoskopspatels 1 ein Maximum erreicht und in distaler Richtung abnimmt.

An das distale Ende des Grundblatts 2 ist der atraumatische Wulst 4 angesetzt, wobei der untere Schenkel 8 des Grundblatts 2 in eine Nut 15 des Wulsts 4 hineinragt. Der Spatelkopf 5 ist hohl ausgebildet und weist Kupplungselemente für eine lösbare mechanische Verbindung mit dem nicht dargestellten Handgriff auf. Auch das Deckblatt 3, der Wulst 4 und der Spatelkopf 5 sind jeweils einstückig ausgebildet und aus Edelstahl gefertigt.

Das Deckblatt 3 schließt mit dem Grundblatt 2 einen längserstreckten Hohlraum 16 ein (s. Fig. 3a, 3b), der distalseitig in einer Öffnung 17 endet. Am proximalen Ende 6 ist der Hohlraum 16 durch eine einstückig mit dem Deckblatt 3 ausgeführte Querwand dicht abgeschlossen, steht jedoch über einen Durchbruch des Grundblatts 2 mit dem Innenraum des hohl ausgebildeten Spatelkopfs 5 in Verbindung (in den Figuren nicht gezeigt).

In den Figuren 3a und 3b ist der Laryngoskopspatel in einem Querschnitt dargestellt, wobei sich die Querschnittsdarstellung etwa auf die Mitte des Laryngoskopspatels 1 bezieht, d.h. der Laryngoskopspatel 1 ist etwa bei seiner halben Länge in Querrichtung geschnitten. In Fig. 3a und 3b ist der Hohlraum 16 zu erkennen, der zwischen dem Grundblatt 2 und dem Deckblatt 3 gebildet wird und der zur Aufnahme optischer und/oder elektronischer Bauelemente dient. Das Deckblatt 3 weist Fügebereiche auf, die durch die untere Kante 12 und die Seitenkante 14 gebildet werden. Ein Teilbereich der Oberseite des unteren Schenkels 8 und der Übergang vom mittleren Schenkel 9 in den oberen Schenkel 10 stellen die Fügebereiche des Grundblatts 2 dar, die mit den Fügebereichen des Deckblatts 3 zusammen wirken, um Lötspalte 18, 19 zu bilden, in denen Lötnähte 20, 21 erzeugt werden, durch die das Deckblatt 3 mit dem Grundblatt 2 fest verbunden wird (s. Fig. 3b). Dies wird im Folgenden näher beschrieben.

Gemäß einem Ausführungsbeispiel des erfindungsgemäßen Verfahrens werden zur Herstellung des Laryngoskopspatels 1 zunächst das Grundblatt 2, das Deckblatt 3, der Wulst 4 und der Spatelkopf 5 bereitgestellt. Diese werden sodann durch Laser-Punktschweißen derart miteinander verbunden, dass diese in der in den Figuren gezeigten Weise angeordnet sind, ohne jedoch schon durch Lötnähte miteinander verbunden zu sein, sondern zwischen sich jeweils einen Lötspalt 18, 19 bilden. Im nächsten Schritt wird ein Lotmaterial 22, nämlich Eisenbasislot in pastöser Form, in einer jeweils zur Erstellung einer Lötnaht notwendigen Menge mit einer Dosiereinrichtung mittels einer gekrümmten Spritzennadel durch die Öffnung 17 in den Hohlraum 16 eingebracht und auf die Innenseiten des Grundblatts 2 und des Deckblatts 3 angrenzend an die Lötspalte 18, 19 aufgetragen (s. Fig. 3a). Hierfür ist beispielsweise das Eisenbasislot ML 7813/S der Fa. Innobraze (Esslingen, DE) geeignet. Das Lotmaterial 22 bildet jeweils einen Wulst, der von innen an den jeweiligen Lötspalt 18, 19 angrenzt und der an den Innenwänden beider Bauteile, die zwischen sich den jeweiligen Lötspalt 18, 19 bilden, anliegt. Ebenso wird das Eisenbasislot in die Nut 15 und in den Spatelkopf 5 benachbart zu einem zwischen dem Spatelkopf 5 und der Unterseite des Grundblatts 2 gebildeten Lötspalt eingebracht. Hierfür ist beispielsweise eine Menge von 0,8g bis 0,9g für die Lötnähte 20, 21, die das Grundblatt 2 und das Deckblatt 3 miteinander verbinden, sowie von 0,5g bis 0,6g für die Lötnaht 24 des Spatelkopfs 5 erforderlich.

Dieser Lötverbund wird sodann in einen Ofen eingebracht und auf eine Löttemperatur von etwa 1120°C erwärmt. Das Eisenbasislot verflüssigt sich dabei und fließt aufgrund der Kapillarwirkung in die zwischen den miteinander zu verbindenden Bauteilen gebildeten Lötspalte 18, 19. Beim nachfolgenden Abkühlen, das beispielsweise innerhalb einer oder weniger Stunden erfolgen kann, erstarrt das Eisenbasislot in den Lötspalten 18, 19 und bildet die Lötnähte 20, 21, um dadurch eine feste, haltbare und dichte Verbindung zwischen den miteinander zu verbindenden Bauteilen zu schaffen. Im gleichen Arbeitsschritt entstehen die Lötnähte 23, 24, die den distalen Wulst 4 bzw. den Spatelkopf 5 mit dem Grundblatt 2 verbinden. Nach erfolgter Abkühlung wird die obere Lötnaht 21 zwischen dem zweiten Schenkel 13 des Deckblatts 3 und dem Übergang vom mittleren Schenkel 9 zum oberen Schenkel 11 des Grundblatts 2 nochmals überschweißt, um die Festigkeit der Verbindung zu erhöhen, und anschließend geglättet.

Der entstehende Zusammenbau wird in weiteren Herstellungsschritten des Laryngoskopspatels mit weiteren Bauteilen versehen. Insbesondere werden optische und elektronische Bauelemente in den Hohlraum 16 eingeführt. So werden in den Hohlraum 16 Lichtleiter zur Weiterleitung von mittels einer externen Lichtquelle erzeugtem Beleuchtungslicht zur distalseitigen Öffnung 17 des Hohlraums 16 oder elektrische Leitungen zur Versorgung einer im Bereich der Öffnung 17 angeordneten Lichtquelle eingeführt. Im Bereich der Öffnung 17 können in den Hohlraum 16 eine Beobachtungsoptik und ein elektronischer Bildaufnehmer eingesetzt werden. Weiter können in den Hohlraum 16 elektrische Leitungen zur Versorgung des elektronischen Bildaufnehmers und zur Signalübertragung oder auch ein Bildleiter zur Weiterleitung eines aufgenommenen Bilds eingeführt werden.

Die Lichtleiter und/oder elektrischen Leitungen sind proximalseitig durch eine nicht in den Figuren dargestellte Öffnung des Grundblatts 2 bis in den Spatelkopf 5 geführt, in den Kupplungselemente zum Anschluss an entsprechende Lichtleiter bzw. elektrische Leitungen, die innerhalb des Handgriffs verlaufen, eingesetzt werden. Über diese werden die elektronischen Bauteile mit elektrischer Energie versorgt und das aufgenommene Bild des Mund-Rachenraums des Patienten zu einer externen Anzeigeeinrichtung weitergeleitet.

Die Öffnung 17 kann beispielsweise durch ein transparentes Fenster oder durch ein Videomodul, das die Beobachtungsoptik und den elektronischen Bildaufnehmer enthält, verschlossen werden. Am proximalen Ende wird der Durchgang zwischen dem Hohlraum 16 und dem Spatelkopf 5 ebenfalls dampfdicht verschlossen. Da die Lötnähte glatt und dampfdicht sind, sind die im Hohlraum 16 angeordneten optischen und elektronischen Bauelemente gegen das Eindringen von Dampf beim Autoklavieren geschützt.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Laryngoskopspatel
- 2: Grundblatt
- 3: Deckblatt
- 4: Wulst
- 5: Spatelkopf
- 6: Proximales Ende
- 7: Distales Ende
- 8: Unterer Schenkel
- 9: Mittlerer Schenkel
- 10: Oberer Schenkel
- 11: Erster Schenkel
- 12: Unterkante
- 13: Zweiter Schenkel
- 14: Seitenkante
- 15: Nut
- 16: Hohlraum
- 17: Öffnung
- 18: Lötspalt
- 19: Lötspalt
- 20: Lötnaht
- 21: Lötnaht
- 22: Lotmaterial
- 23: Lötnaht
- 24: Lötnaht

## Patentansprüche

1. Verfahren zum Fügen mindestens zweier Bauteile eines invasiven medizinischen Instruments, wobei die mindestens zwei Bauteile zum Ausbilden mindestens eines Lötspalts (18, 19) zwischen einander zugeordneten Fügebereichen der Bauteile gehalten werden, ein Lotmaterial (22) zum Ausfüllen des mindestens einen Lötspalts (18, 19) angeordnet wird und die Anordnung aus den mindestens zwei Bauteilen und dem Lotmaterial (22) auf eine Löttemperatur des Lotmaterials (22) erwärmt wird, **dadurch gekennzeichnet, dass** das Lotmaterial (22) ein Eisenbasislot ist.

2. Verfahren nach Anspruch 1, **dadurch gekennzeichnet, dass** die mindestens zwei Bauteile zum Ausbilden des mindestens einen Lötspalts (18, 19) vor dem Erwärmen mittels Laserschweißen miteinander verbunden werden.

3. Verfahren nach Anspruch 1 oder 2, **dadurch gekennzeichnet, dass** zwischen den mindestens zwei Bauteilen ein Hohlraum (16) gebildet wird und dass das Lotmaterial (22) innerhalb des Hohlraums (16) angeordnet wird.

4. Verfahren nach einem der vorhergehenden Ansprüche, **dadurch gekennzeichnet, dass** das Lotmaterial (22) in pastöser Form angeordnet wird.

5. Verfahren nach einem der vorhergehenden Ansprüche, **dadurch gekennzeichnet, dass** mindestens eine durch das Lotmaterial (22) und das Erwärmen gebildete Lötnaht (20, 21) fluiddicht ausgebildet ist.

6. Verfahren nach einem der vorhergehenden Ansprüche, **dadurch gekennzeichnet, dass** mindestens eine durch das Lotmaterial (22) und das Erwärmen gebildete Lötnaht (21) überschweißt wird.

7. Verfahren nach Anspruch 6, **dadurch gekennzeichnet, dass** die mindestens eine überschweißte Lötnaht (21) geglättet wird.

8. Verwendung eines Eisenbasislots zum Fügen mindestens zweier Bauteile eines Laryngoskopspatels (1) gemäß einem Verfahren nach einem der Ansprüche 1 bis 7.

9. Invasives medizinisches Instrument, insbesondere Laryngoskopspatel (1), mit mindestens zwei Bauteilen, **dadurch gekennzeichnet, dass** die mindestens zwei Bauteile durch ein Verfahren gemäß einem der Ansprüche 1 bis 7 miteinander verbunden worden sind.

10. Invasives medizinisches Instrument gemäß Anspruch 9, **dadurch gekennzeichnet, dass** das medizinische Instrument ein Laryngoskopspatel (1) ist, der ein Grundblatt (2) und ein Deckblatt (3) aufweist, die einen längserstreckten Hohlraum (16) ausbilden, der durch mindestens eine durch das Lotmaterial (22) gebildete Lötnaht (20, 21) fluiddicht begrenzt ist.

## Claims

1. Method for joining at least two components of an invasive medical instrument, wherein the at least two components are held so as to form at least one soldering gap (18, 19) between mutually assigned joining areas of the components, a solder material (22) is arranged for filling the at least one soldering gap (18, 19), and the arrangement of the at least two components and of the solder material (22) is heated to a soldering temperature of the solder material (22), **characterized in that** the solder material (22) is an iron-based solder.

2. Method according to Claim 1, **characterized in that** the at least two components for forming the at least one soldering gap (18, 19) are connected to each other by means of laser welding before the heating.

3. Method according to Claim 1 or 2, **characterized in that** a cavity (16) is formed between the at least two components, and **in that** the solder material (22) is arranged inside the cavity (16).

4. Method according to one of the preceding claims, **characterized in that** the solder material (22) is arranged in pasty form.

5. Method according to one of the preceding claims, **characterized in that** at least one soldering seam (20, 21) formed by the solder material (22) and the heating is fluid-tight.

6. Method according to one of the preceding claims, **characterized in that** at least one soldering seam (21) formed by the solder material (22) and the heating is overwelded.

7. Method according to Claim 6, **characterized in that** the at least one overwelded soldering seam (21) is smoothed.

8. Use of an iron-based solder to join at least two components of a laryngoscope spatula (1) according to a method according to one of Claims 1 to 7.

9. Invasive medical instrument, in particular a laryngoscope spatula (1), with at least two components, **characterized in that** the at least two components have been connected to each other by a method according to one of Claims 1 to 7.

10. Invasive medical instrument according to Claim 9, **characterized in that** the medical instrument is a laryngoscope spatula (1) having a base blade (2) and a cover blade (3) which form an elongate cavity (16), which is delimited in a fluid-tight manner by at least one soldering seam (20, 21) formed by the solder material (22).

## Revendications

1. Procédé d'assemblage d'au moins deux composants d'un instrument médical invasif,
les au moins deux composants étant retenus de manière à réaliser au moins un interstice de brasage (18, 19) entre des régions d'assemblage des composants associées l'une à l'autre, une matière de brasage (22) étant prévue pour remplir l'au moins un interstice de brasage (18, 19) et l'agencement constitué des au moins deux composants et de la matière de brasage (22) étant chauffé à une température de brasage du matériau de brasage (22), **caractérisé en ce que** le matériau de brasage (22) est une brasure à base de fer.

2. Procédé selon la revendication 1, **caractérisé en ce que** les au moins deux composants sont connectés l'un à l'autre au moyen d'un soudage laser avant le chauffage afin de réaliser l'au moins un interstice de brasage (18, 19).

3. Procédé selon la revendication 1 ou 2, **caractérisé en ce qu'**entre les au moins deux composants est formée une cavité (16) et **en ce que** la matière de brasage (22) est disposée à l'intérieur de la cavité (16).

4. Procédé selon l'une quelconque des revendications précédentes, **caractérisé en ce que** la matière de brasage (22) est prévue sous forme de pâte.

5. Procédé selon l'une quelconque des revendications précédentes, **caractérisé en ce qu'**au moins un joint brasé (20, 21) formé à partir de la matière de brasage (22) et du chauffage est réalisé de manière étanche aux fluides.

6. Procédé selon l'une quelconque des revendications précédentes, **caractérisé en ce qu'**au moins un joint brasé (21) formé à partir de la matière de brasage (22) et du chauffage est soudé par-dessus.

7. Procédé selon la revendication 6, **caractérisé en ce que** l'au moins un joint brasé (21) soudé par-dessus est lissé.

8. Utilisation d'une brasure à base de fer pour l'assemblage d'au moins deux composants d'une spatule de laryngoscope (1) selon un procédé selon l'une quelconque des revendications 1 à 7.

9. Instrument médical invasif, en particulier spatule de laryngoscope (1), comprenant au moins deux composants, **caractérisé en ce que** les au moins deux composants ont été assemblés l'un à l'autre par un procédé selon l'une quelconque des revendications 1 à 7.

10. Instrument médical invasif selon la revendication 9, **caractérisé en ce que** l'instrument médical est une spatule de laryngoscope (1) qui présente une lame de base (2) et une lame supérieure (3) qui constituent une cavité allongée (16) qui est limitée de manière étanche aux fluides par au moins un joint brasé (20, 21) formé par la matière de brasage (22).
